## Europäisches Patentamt

## European Patent Office

## Office européen des brevets

(19)

(11) Veröffentlichungsnummer: **0 349 735 B1**

# EUROPÄISCHE PATENTSCHRIFT

(12)

(45) Veröffentlichungstag der Patentschrift: **23.12.92**

(21) Anmeldenummer: **89108512.8**

(22) Anmeldetag: **11.05.89**

(51) Int. Cl.⁵: **A61L 2/00**

(54) Verwendung von Alkalisalzen der Sorbinsäure bei der Hitzesterilisierung von Polyethylenoxid enthaltenden wässerigen Lösungen.

(30) Priorität: **08.07.88 DE 3823265**

(43) Veröffentlichungstag der Anmeldung:
**10.01.90 Patentblatt 90/02**

(45) Bekanntmachung des Hinweises auf die
Patenterteilung:
**23.12.92 Patentblatt 92/52**

(84) Benannte Vertragsstaaten:
**AT BE CH FR GB GR LI NL**

(56) Entgegenhaltungen:
**FR-A- 2 073 437
US-A- 3 227 652
US-A- 4 058 213
US-A- 4 409 205**

(73) Patentinhaber: **Dr. Thilo & Co. GmbH
Rudolf-Diesel-Ring 21
W-8029 Sauerlach(DE)**

(72) Erfinder: **Tschöpe, Michael, Dr.
Bellizonastrasse 1
W-8000 München 71(DE)**
Erfinder: **Kanduth, Monika
Spixstrasse 10
W-8000 München 90(DE)**

(74) Vertreter: **von Füner, Alexander, Dr. et al
Patentanwälte v. Füner, Ebbinghaus, Finck
Mariahilfplatz 2 & 3
W-8000 München 90(DE)**

**Beschreibung**

Die vorliegende Erfindung betrifft die sterile Herstellung von Lösungen, welche mit den Geweben des Körpers, insbesondere mit den Geweben des Auges, in Berührung kommen.

Die erfindungsgemäß erhaltenen Lösungen können als Katheterschmiermittel, zur Befeuchtung von Schleimhäuten des menschlichen und tierischen Körpers sowie als Tragerlösunger für ophthalmologische Präparate dienen. Darüber hinaus eignen sich diese Lösungen auch als Tränenersatzmittel sowie als Benetzungsmittel für Contactlinsen.

Die Anwendung der erfindungsgemäß erhaltenen Lösungen am menschlichen oder tierischen Auge ist hierbei besonders bevorzugt.

Augentropfenlösungen, die in Kombination mit Contactlinsen verwendet werden können oder als Tränenflüssigkeitsersatz dienen, sind bekannt. Neben üblichen Formulierungshilfsstoffen für Augentropfen enthalten die Lösungen als wesentliche Bestandteile polymere Verbindungen wie Methylcellulose, Carboxymethylcellulose, Hydroxypropylmethylcellulose, Hydroxyethylcellulose, Polyvinylpyrrolidon, Polyvinylalkohol sowie Polyethylenoxid mit einem Molekulargewicht über 100.000 (F. J. Holly, Contact Lens. 4, 14 (1978). Im allgemeinen dient die Anwesenheit der Polymeren in der Augentropfenlösung dem Zweck, die Viskosität der Lösung zu erhöhen. Durch die erhöhte Viskosität soll sowohl die Verweildauer der Lösung am Auge verlängert als auch eine Abmilderung von Reizungen bewirkt werden, die z.B. durch das Tragen von Contactlinsen hervorgerufen werden können.

In der DE-A-20 51 369 wird eine ophthalmische Lösung beschrieben, die dadurch gekennzeichnet ist, daß sie in einer wässerigen Lösung ein Ethylenoxidpolymerisat mit einem Molekulargewicht von mindestens etwa 100.000 in Kombination mit einem Polyalkylenglycol enthält. Der Molekulargewichtsbereich des verwendeten Polyethylenoxids ist sehr weit und kann von 100.000 bis zu mehreren Millionen, wie beispielsweise 5 Mitlionen oder mehr reichen. Es werden dabei höhermolekulare Produkte bevorzugt, wobei sich ein Bereich von 4 Millionen Molekulargewicht als besonders zweckmäßig erwiesen hat. Ethylenoxidpolymerisate mit diesem Molekulargewicht sind unter der Bezeichnung Polyox WSR 301® bekannt. Ethylenoxidpolymerisate mit einem Molekulargewicht bis zu 5 Millionen weisen einen ausgezeichneten Schmiereffekt auf, was aus entsprechenden Patentschriften (US-PS'en 3 227 652 und 3 699 057) hervorgeht. Die beiden letztgenannten Patentschriften beziehen sich auf Schmiermittel für einen Einsatz im technischen Bereich. Im Gegensatz zu diesen technischen Produkten müssen Lösungen zur Anwendung am Auge oder zur Verwendung als Contactlinsenbenetzunglösung steril sein. Eine Sterilisation im Autoklaven für 20 Minuten bei 121°C gilt als sicherste Sterilisationsmethode. Wässerige Lösungen von Ethylenoxidpolymerisaten mit Molekulargewichten bis zu 5 Millionen lassen sich jedoch nur unter Schwierigkeiten autoklavieren. Aus der DE-A-20 51 369 geht hervor, daß das Polymer bei höheren Temperaturen ausfallen kann und nach Abkühlung nur sehr langsam wieder in Lösung geht.

Ein wesentliches Problem ist jedoch der starke Viskositätsabfall, welcher bei der Sterilisierung von Ethylenoxidpolymerisaten mit Molekulargewichten bis zu 5 Millionen infolge der Hitzeeinwirkung auftreten kann. Die durch höhere Temperaturen hervorgerufene Abnahme der Viskosität ist auch für andere Polymere, z.B. Cellulosederivate, bekannt. Ursache dieser Viskositätsabnahme ist ein teilweiser thermischer Abbau der Polymerketten, der zu kürzeren Polymerketten und demzufolge zu einer geringeren Viskosität führt. Im Falle von Ethylenoxidpolymerisaten mit Molekulargewichten bis zu 5 Millionen bewirkt der Einfluß höherer Temperaturen, wie sie beim Sterilisieren auftreten, neben dem Abfall der Viskosität auch eine dramatische Verschlechterung der Schmiereigenschaften. Während eine angestrebte Viskosität durch einen entsprechenden Mehreinsatz der Polymere, welcher die Viskositätsabnahme während der Sterilisation berücksichtigt, erzielt werden kann, ist eine solche Zubereitung hinsichtlich der Schmierwirkung nicht mit einer nichtautoklavierten Zubereitung gleicher Viskosität zu vergleichen. Die ausgezeichnete Schmierwirkung von Ethylenoxidpolymerisaten mit Molekulargewichten bis zu 5 Millionen ist im wesentlichen durch die langen Polymerketten bedingt. Aufgrund des derzeitigen Standes der Technik ist eine Hitzesterilisation ohne Zerstörung der Polymerketten, kenntlich an einem deutlichen Viskositätsabfall während der Sterilisation, nicht möglich.

Neben der bevorzugten Methode der Hitzesterilisation können zur Sterilmachung von wässerigen Lösungen auch andere Methoden eingesetzt werden, welche auf höhere Temperatur verzichten. So kann z.B. bei niedrigviskosen Flüssigkeiten eine Sterilfiltration vorgenommen werden. Im Falle von Lösungen mit Ethylenoxidpolymerisaten mit Molekulargewichten bis 5 Millionen ist diese Methode jedoch nicht anwendbar, da die Polymere die Filter verstopfen. Eine weitere Methode ist die in der DE-A-20 51 369 erwähnte Begasung mit Ethylenoxid. Aufgrund des derzeitigen Standes der wissenschaftlichen Erkenntnisse stellt eine Begasung mit Ethylenoxid jedoch ein erhebliches Risiko dar. Wegen der Reaktivität des Ethylenoxids sind Reaktionen mit dem Sterilisiergut möglich. Des weiteren können im Sterilisiergut toxische Abbauprodukte des Ethylenoxids zurückbleiben.

Aufgrund der oben beschriebenen Probleme ist es zur Zeit nicht möglich, wässerige Lösungen von Ethylenoxidpolymerisaten mit Molekulargewichten bis zu 5 Millionen unter wesentlicher Erhaltung der Viskosität und vor allem der Schmiereigenschaften, mit Hitze zu sterilisieren.

Aufgabe der vorliegenden Erfindung war es daher, einen Stoff zu finden, welcher mit menschlichen oder tierischen Geweben verträglich ist und welcher die Viskosität sowie den Schmiereffekt wässeriger Lösungen von Polyethylenoxid mit Molekulargewichten von etwa 100.000 bis zu 5 Millionen trotz Hitzesterilisation bei höheren Temperaturen, z.B. bei 121°C, weitgehend zu stabilisieren in der Lage ist.

Diese Aufgabe wird wie aus dem vorstehenden Anspruch ersichtlich gelöst.

Es hat sich überraschenderweise gezeigt, daß wässerige Lösungen mit einem Gehalt an Polyethylenoxid mit einem Molekulargewicht von über 100.000 bis zu 5 Millionen in Anwesenheit von Alkalisalzen der Sorbinsäure ohne relevante Viskositätsverluste und Reduzierung der Schmierwirkung hitzesterilisiert werden können. Der stabilisierende Einfluß der Salze der Sorbinsäure bei der Hitzesterilisation war aus dem Stand der Wissenschaft und Technik nicht abzuleiten.

Die erfindungsgemäßen Lösungen können neben Alkalisalzen der Sorbinsäure weitere mit den Körpergeweben verträgliche Substanzen enthalten, die der pH-Einstellung oder Pufferung, der Isotonisierung, der Konservierung, der Viskositätserhöhung oder der Benetzung dienen.

Diese Substanzen sind dem Fachmann an sich geläufig. Sie umfassen u.a. anorganische Salze wie Natriumchlorid, Kaliumchlorid und Kaliumnitrat sowie Zucker oder Zuckeralkohole wie Sorbit, xylit, Mannit oder Glucose zur Isotonisierung.

Zur pH-Einstellung auf Werte zwischen 5 und 9 sind beispielsweise Natronlauge, Kalilauge, Salzsäure oder Schwefelsäure in geeigneten Verdünnungen verwendbar.

Zur Pufferung können herkömmliche dem Fachmann geläufige Puffersysteme wie z.B. Boratpuffer, Phosphatpuffer oder Citratpuffer eingesetzt werden.

Als viskositätserhöhende Stoffe können in den erfindungsgemäß erhaltenen Lösungen Methylcellulose, Carboxymethylcellulose, Hydroxypropylmethylcellulose, Hydroxyethylcellulose, Polyvinylpyrrolidon sowie Polyvinylalkohol Verwendung finden.

Als herkömmliche Konservierungsmittel für die erfindungsgemäßen Lösungen eignen sich z.B. Thiomersal, Phenylquecksilbersalze, Parabene sowie quartäre Ammoniumverbindungen wie z.B. Benzalkoniumchlorid in adäquater Dosierung.

Die Alkalisorbate werden vorzugsweise in einer Menge von 0,01 bis 10 % (G/V) eingesetzt, wobei 0,1 bis 0,5 % (G/V) bevorzugt ist. Bevorzugte Alkalisorbate sind Kaliumsorbat und Natriumsorbat, insbesondere Kaliumsorbat.

Versuch 1

Lösungen mit 0,25 % (G/G) Polyox WSR 301 (Polyethylenoxid, Molekulargewicht 4 Millionen) werden unter Zusatz unterschiedlicher in Tabelle 1 angeführter Substanzen 30 Minuten bei 121°C hitzesterilisiert. Vor und nach der Hitzesterilisation erfolgt die Bestimmung der viskosität (Kapillarviskosimeter, 20° C) sowie eine Beurteilung der Schmierwirkung. Die einzelnen Ansätze, sowie die Ergebnisse sind in Tabelle 1 dargestellt.

Aus dieser Tabelle geht deutlich hervor, daß eine wässerige Lösung von Polyethylenoxid mit einem Molekulargewicht von 4 Millionen durch Hitzesterilisation einen großen Viskositätsverlust erleidet. Die Schmierwirkung ist ebenfalls drastisch reduziert (siehe Ansatz 1). Der Zusatz von organischen Säuren wie Sorbinsäure, Citronensäure und Weinsäure (Ansätze 2, 5 und 6) ist nicht in der Lage, die Polymerdegradation zu verhindern.

Die Kalium- und Natriumsalze von Citronensäure und Weinsäure (Ansätze 7 bis 10) bewirken ebenfalls keine Stabilisierung der Viskosität sowie der Schmierwirkung. Auch der Zusatz von anorganischen Kalium- und Natriumsalzen zeigt keinen Erfolg (Ansätze 3 und 4).

Lediglich der Zusatz von Kaliumsorbat ist überraschenderweise in der Lage, die wichtigen Produkteigenschaften auch nach der Hitzesterilisation aufrechtzuerhalten (siehe Ansatz 11).

Versuch 2

Wässerige Lösungen von 0,25 % (G/G) Polyethylenoxid mit einem Molekulargewicht von 4 Millionen wurden mit Kaliumsorbat, mit Sorbinsäure und Natriumchlorid sowie mit Kaliumchlorid und Sorbinsäure versetzt und für 30 Minuten bei 121°C hitzesterilisiert. Durch pH-Einstellung auf 7 bis 8 wurden die Alkalisalze der Sorbinsäure im Falle der letzteren beiden Ansätze erst in Lösung erzeugt. Die Ergebnisse sind in Tabelle 2 dargestellt.

Die Resultate zeigen erneut den stabilisierenden Effekt von Alkalisalzen der Sorbinsäure auf die Viskosität und die Schmierwirkung bei der Hitzesterilisation wässeriger Lösungen von Polyethylenoxid mit einem Molekulargewicht von 4 Millionen. Dabei ist es unerheblich, ob die Salze als solche zugesetzt werden, oder erst durch Neutralisation mit einer Alkalilauge in der Lösung erzeugt werden.

Versuch 3

Wässerige Lösungen von Polyethylenoxid mit einem Molekulargewicht von 4 Millionen in unterschiedlicher Konzentration, wurden ohne Zusatz von Alkalisalzen der Sorbinsäure autoklaviert.

Die Ergebnisse sind in Tabelle 3 dargestellt. Die Resultate zeigen, daß auch durch sehr hohe Konzentrationen von Polyethylenoxid mit einem Molekulargewicht von 4 Millionen keine hitzesterilisierte Lösung erhalten werden kann, welche einen sehr guten Schmiereffekt aufweist. Aus Ansatz 19 ist zu ersehen, daß die Viskosität alleine noch keine sehr gute Schmierwirkung bedingt (Vergleiche zu Tabelle 2).

Versuch 4

Die Lösungen des Versuchs 3 wurden unter Zusatz von jeweils 0,2 % (G/G) Kaliumsorbat erneut hergestellt und bei 121°C für 30 Minuten hitzesterilisiert. Die Ergebnisse sind in Tabelle 4 dargestellt.

Die Resultate zeigen, daß in allen Fällen die Schmierwirkung der autoklavierten Proben beibehalten wird. Der Viskositätsabfall während der Hitzesterilisation wird gegenüber den Ergebnissen des Versuchs 3 sehr stark reduziert.

Versuch 5

Wässerige Lösungen mit 0,25 % (G/G) Polyethylenoxid mit einem Molekulargewicht von 4 Millionen wurden mit unterschiedlichen Mengen Kaliumsorbat versetzt und vor sowie nach der Hitzesterilisation hinsichtlich Viskosität und Schmierwirkung untersucht. Die Ergebnisse sind in Tabelle 5 aufgeführt.

Die Resultate zeigen, daß Konzentrationen über 0,01 % Kaliumsorbat die Viskosität und den Schmiereffekt von wässerigen Lösungen mit Polyethylenoxid nach der Hitzesterilisation weitgehend erhalten.

Die Versuche 1 bis 5 dienen lediglich der Verdeutlichung der Erfindung, ohne aber den Umfang der Erfindung zu beschränken. So wurden die Versuche zum Großteil mit Kaliumsorbat als erfindungsgemäßem Stabilisator durchgeführt, wobei auch Natriumsorbat und andere Alkalisalze der Sorbinsäure zu ähnlichen Ergebnissen führen. Ebensowenig soll durch die aufgeführten Versuche die Erfindung auf den direkten Zusatz der Alkalisalze der Sorbinsäure eingeengt werden. Gleiche Resultate werden erhalten bei der Bildung der Salze in situ, z.B. bei Zugabe von Sorbinsäure und deren Neutralisation in der Lösung durch Zugabe basischer Substanzen, welche Alkalimetallionen enthalten. Beispielsweise seien Natriumhydroxid und Kaliumhydroxid genannt. Im Rahmen der Erfindung liegt ebenfalls die Verwendung von Puffersubstanzen, welche Alkaliionen enthalten und welche in der Lage sind, die polyethylenoxidhaltige Lösung bei Anwesenheit von Sorbinsäure auf pH 5,0 bis 9,0 abzupuffern.

Im weitesten Sinne umfaßt die Erfindung alle dem Fachmann an sich geläufigen Methoden, welche dazu führen, daß Alkalisalze der Sorbinsäure, unabhängig von deren Dissoziationsgrad, in einer polyethylenoxidhaltigen wässerigen Lösung enthalten sind.

Tabelle 1

| Versuch Nr. | Zugesetzter Stoff, % (G/G) | Vor dem Autoklavieren | | Nach dem Autoklavieren | |
|---|---|---|---|---|---|
| | | Viskosität (mPa.s) | Schmiereffekt | Viskosität (mPa.s) | Schmiereffekt |
| 1 | ohne | 7,6 | + + + | 1,6 | - |
| 2 | Sorbinsäure 0,1% | 6,9 | + + + | 1,6 | - |
| 3 | KCl 0,2% | 7,8 | + + + | 1,9 | - |
| 4 | NaCl 0,2% | 7,9 | + + + | 1,5 | - |
| 5 | Weinsäure 0,2% | 4,2 | + + | 1,3 | - |
| 6 | Citronensäure 0,2% | 4,1 | + + | 1,3 | - |
| 7 | Tri-Natrium-Citrat x 2 $H_2O$ 0,2% | 7,7 | + + + | 2,0 | - |
| 8 | Di-Natrium-tartrat 0,2% | 7,5 | + + + | 1,7 | - |
| 9 | Di-Kalium-tartrat 0,2% | 8,0 | + + + | 1,7 | - |
| 10 | Tri-Kalium-Citrat Monohydrat 0,2% | 8,4 | + + + | 2,3 | + |
| 11 | Kaliumsorbat 0,2 % | 8,2 | + + + | 7,3 | + + + |

Einfluß unterschiedlicher Substanzen auf die Viskosität und den Schmiereffekt wäßriger Lösungen von Polyox WSR 301, 0,25% (G/G) vor und nach der Hitzesterilisation.
Schmiereffekt: + + + sehr gut, + + gut, + mäßig,-keiner

Tabelle 2

| Versuch Nr. | Zugesetzter Stoff, % (G/G) | Vor dem Autoklavieren | | Nach dem Autoklavieren | |
|---|---|---|---|---|---|
| | | Viskosität | Schmier effekt | Viskosität | Schmiereffekt |
| 12 | Kaliumsorbat 0,2% | 7,8 | + + + | 6.7 | + + + |
| 13 | Sorbinsäure 0,1% NaCl 0,2% pH 7,5 mit NAOH | 7,6 | + + + | 6,7 | + + + |
| 14 | Sorbinsäure 0,1% KCl 0,2% pH 7,5 mit NAOH | 7,6 | + + + | 5,7 | + + + |

Einfluß unterschiedlicher Stoffe und Stoffgemische auf die Viskosität und den Schmiereffekt wäßriger Lösungen von 0,25% (G/G) Polyethylenoxid mit einem MG von 4 Mio. vor und nach dem Autoklavieren
Schmiereffekt: + + + sehr gut
+ + gut
+ mäßig
- keiner

Tabelle 3

| Vers. Nr. | Konz. an Polyethylenoxid MG 4 Mio. % (G/G) | Vor dem Autoklavieren | | Nach dem Autoklavieren | |
|---|---|---|---|---|---|
| | | Viskosität (mPa•s) | Schmiereffekt | Viskosität (mPa•s) | Schmiereffekt |
| 15 | 0,05% | 1,8 | + | 1,3 | - |
| 16 | 0,25% | 7,8 | + + + | 1,9 | - |
| 17 | 0,50% | 36,6 | + + + | 3,1 | - |
| 18 | 0,75% | 92,9 | + + + | 5,4 | + |
| 19 | 1,00% | 381 | + + + | 12,6 | + |

Einfluß der Hitzesterilisation (30 Min./ 121°C) auf die Viskosität und den Schmiereffekt wäßriger Lösungen mit unterschiedlichen Konzentrationen an Polyethylenoxid mit einem MG von 4 Mio.
Schmiereffekt: + + + sehr gut
+ + gut
+ mäßig
- keiner

Tabelle 4

| Vers. Nr. | Zusammensetzung % (G/G) Wasser ad 100,0% | Vor dem Autoklavieren | | Nach dem Autoklavieren | |
|---|---|---|---|---|---|
| | | Viskosität (mPa•s) | Schmiereffekt | Viskosität (mPa•s) | Schmiereffekt |
| 20 | 0,05% Polyox WSR 301 0,2% Kaliumsorbat | 1,9 | + | 1,7 | + |
| 21 | 0,25% Polyox WSR 301 0,2% Kaliumsorbat | 7,8 | + + + | 6,6 | + + + |
| 22 | 0,5 % Polyox WSR 301 0,2% Kaliumsorbat | 31,9 | + + + | 26,2 | + + + |
| 23 | 0,75% Polyox WSR 301 0,2% Kaliumsorbat | 117 | + + + | 109 | + + + |
| 24 | 1,0% Polyox WSR 301 0,2% Kaliumsorbat | 514 | + + + | 446 | + + + |

Einfluß von 0,2% Kaliumsorbat auf die Viskosität und den Schmiereffekt von wäßrigen Lösungen mit unterschiedlichen Konzentrationen an Polyox WSR 301 während der Autoklavierung bei 121°C / 30 Min.
Schmiereffekt: + + + sehr gut
+ + gut
+ mäßig
- keiner

Tabelle 5

| Vers. Nr. | Konz. Kaliumsorbat % (G/G) | Vor dem Autoklavieren | | Nach dem Autoklavieren | |
|---|---|---|---|---|---|
| | | Viskosität (mPa•s) | Schmiereffekt | Viskosität (mPa•s) | Schmiereffekt |
| 25 | 0,01% | 7,5 | + + + | 1,9 | - |
| 26 | 0,1 % | 8,1 | + + + | 6,5 | + + + |
| 27 | 0,2 % | 8,2 | + + + | 6,7 | + + + |
| 28 | 0,4 % | 8,4 | + + + | 7,4 | + + + |
| 29 | 1,0 % | 8,4 | + + + | 7,4 | + + + |
| 30 | 1,5 | 7,5 | + + + | 6,9 | + + + |
| 31 | 5,0 | 9,3 | + + + | 7,2 | + + + |

Einfluß von unterschiedlichen Mengen Kaliumsorbat auf die Viskosität und den Schmiereffekt wäßriger Lösungen mit 0,25% (G/G) Polyethylenoxid mit einem MG von 4 Mio. während der Hitzesterilisation 121°C (30 Min.)
Schmiereffekt: + + + sehr gut
+ + gut
+ mäßig
- keiner

Beispiel 1

Rezeptur:

| | |
|---|---|
| 1. Polyethylenoxid, MG 4 Mio. | 0,175 kg |
| 2. Kaliumsorbat | 0,140 kg |
| 3. NaCl | 0,490 kg |
| 4. Dinatrium-edetat | 0,070 kg |
| 5. dest. Wasser | 69,125 kg |

Die Bestandteile 2-4 werden in einem 100-Liter-Reaktor mit Doppelmantel in Bestandteil 5 gelöst. Mittels eines geeigneten Rührwerks wird Bestandteil 1 anschließend unter Erwärmung auf 60-70°C darin gelöst. Die Lösung wird im Reaktor bei 121°C für 30 Minuten hitzesterilisiert, wobei es zu einer Ausfällung kommt. Durch Rühren während der Abkühlphase geht die Ausfällung jedoch wieder in Lösung.

Man erhält so eine Lösung mit einer Viskosität von 6,8 mPa.s und einem pH von 7,0. Die Lösung ist als Arzneistoffträger für ophthalmologische Präparate sowie als Benetzungslösung für Contactlinsen geeignet.

Beispiel 2

Rezeptur:

| 1. Polyethylenoxid, MG 400.000 | 9,20 kg |
|---|---|
| 2. Sorbinsäure | 0,40 kg |
| 3. 1 N NaOH | 3,60 kg |
| 4. Thiomersal | 0,008 kg |
| 5. NaCl | 3,00 kg |
| 6. dest. Wasser | 383,792 kg |

Die Bestandteile 2 + 5 werden in einem 500-Liter-Reaktor mit Doppelmantel in 380,0 kg Bestandteil 6 gelöst. Der pH-Wert der Lösung wird mit Bestandteil 3 auf pH 7,3 eingestellt. Mittels eines geeigneten Rührwerks wird Bestandteil 1 unter Erwärmung auf 60-70° darin zur Lösung gebracht. Die Lösung wird im Reaktor unter Rühren bei 121°C für 30 Minuten hitzesterilisiert. Das durch die Autoklavierung ausfallende Polymer wird während der Abkühlphase durch Rühren wieder aufgelöst. Bei 30°C wird Bestandteil 4, gelöst in der restlichen Menge Wasser, durch einen Sterilfilter der Porenweite 0,2 μm zugesetzt. Anschlie-ßend wird bis zur Abkühlung auf Raumtemperatur gerührt. Man erhält eine Lösung mit einer Viskosität von 32,3 mPa•s und einem pH von 7,25. Die Lösung ist als Arzneistoffträger für ophthalmologische Präparate mit Langzeitwirkung sowie als Benetzungslösung für Contactlinsen geeignet.

Beispiel 3

Rezeptur:

| 1. Polyethylenoxid, MG 4 Mio. | 2,000 kg |
|---|---|
| 2. Mannit | 18,000 kg |
| 3. Kaliumsorbat | 0,800 kg |
| 4. Thiomersal | 0,008 kg |
| 5. dest. Wasser | 379,192 kg |

In einem 500-Liter-Reaktor mit Rührwerk und Doppelmantel werden Bestandteile 2-3 in 375,0 kg des Wassers (Bestandteil 5) gelöst. Anschließend erffolgt Zugabe des Bestandteils 1 und Auflösung desselben durch Rühren und Erwärmen auf 60-70°C. Die so bereitete Polymerlösung wird für 30 Minuten bei 121°C im Reaktor hitzesterilisiert, wobei das Polymer ausfällt. Der Reaktor wird unter Rühren abgekühlt, wodurch das Polymer wieder in Lösung geht. Bei 30°C wird Bestandteil 4 in der restlichen Menge Wasser gelöst und durch einen Sterilfilter mit einer Porenweite von 0,2 μm zugesetzt. Abschließend wird bis zum Erreichen der Raumtemperatur gerührt.

Man erhält eine Lösung mit einer Viskosität von 26,8 mPa.s und einem pH von 7,4. Die Lösung ist geeignet als Trägerlösung für ophthalmologische Präparate sowie als Tränenersatzmittel.

Beispiel 4

Rezeptur:

| 1. Polyethylenoxid, MG 900.000 | 0,540 kg |
|---|---|
| 2. NaCl | 1,020 kg |
| 3. $NaH_2 PO_4 • 2 H_2 O$ | 0,090 kg |
| 4. $Na_2 HPO_4$ wasserfrei | 0,375 kg |
| 5. Natriumsorbat | 0,450 kg |
| 6. Thiomersal | 0,003 kg |
| 7. dest. Wasser | 147,522 kg |

Die Bestandteile 2-5 werden in einem 150-Liter-Reaktor mit Doppelmantel in 147,0 kg Bestandteil 7 gelöst. Darin wird unter Erwärmung auf 60-70°C Bestandteil 1 unter Rühren zur Auflösung gebracht. Die so bereitete Lösung wird für 30 Minuten bei 121°C hitzesterilisiert, wobei das Polymer ausfällt. Durch Abkühlen und Rühren wird das Polymer anschließend wieder in Lösung gebracht. Bei Abkühlung auf 30°C wird Bestandteil 6, gelöst in der restlichen Menge Bestandteil 7, durch einen Sterilfilter der Porenweite 0,2 μm zugesetzt. Es wird anschließend bis Erreichen der Raumtemperatur gerührt.

Man erhält eine Lösung mit einer Viskosität von 7,6 mPa.s und einem pH von 7,5. Die Lösung ist als Träger für ophthalmologische Präparate, als Tränenersatzflüssigkeit sowie als Contactlinsenbenetzungslösung zu verwenden.

Beispiel 5

Rezeptur:

| 1. Polyethylenoxid, MG 600.000 | 1,00 kg |
|---|---|
| 2. NaCl | 2,72 kg |
| 3. $NaH_2PO_4 \cdot 2\,H_2O$ | 0,24 kg |
| 4. $Na_2HPO_4$ wasserfrei | 1,00 kg |
| 5. Sorbinsäure | 0,44 kg |
| 6. Benzalkoniumchlorid | 0,04 kg |
| 7. dest. Wasser | 394,56 kg |

In einem 500-Liter-Reaktor mit Doppelmantel werden die Beteile 2-6 in Bestandteil 7 unter Rühren gelöst. Bestandteil 1 wird anschließend unter Erwärmung auf 60-70° und Rühren darin zur Lösung gebracht. Die Polymerlösung wird dann 30 Minuten bei 121°C hitzesterilisiert, wobei das Polymer ausfällt. Durch Rühren während der Abkühlphase kann das Polymer wieder in Lösung gebracht werden. Man erhält eine Lösung mit einer Viskosität von 4,0 mPa·s und einem pH von 6,7. Die Lösung ist als Trägerlösung für ophthalmologische Präparate sowie als Benetzungslösung für harte Contactlinsen geeignet.

Beispiel 6

Rezeptur:

| 1. Polyethylenoxid, MG 100.000 | 18,000 kg |
|---|---|
| 2. Mannit | 18,000 kg |
| 3. Kaliumsorbat | 0,800 kg |
| 4. Thiomersal | 0,008 kg |
| 5. dest. Wasser | 363,192 kg |

In einem. 500-Liter-Reaktor mit Rührwerk und Doppelmantel werden Bestandteile 2-3 in 360,0 kg des Wassers (Bestandteil 5) gelöst. Anschließend erfolgt Zugabe des Bestandteils 1 und Auflösung desselben durch Rühren und Erwärmen auf 60-70°C. Die so bereitete Polymerlösung wird für 5 Minuten bei 134°C im Reaktor hitzesterilisiert, wobei das Polymer ausfällt. Der Reaktor wird unter Rühren abgekühlt, wodurch das Polymer wieder in Lösung geht. Bei 30°C wird Bestandteil 4 in der restlichen Menge Wasser gelöst und durch einen Sterilfilter mit einer Porenweite von 0,2 μm zugesetzt. Abschließband wird bis zum Erreichen der Raumtemperatur gerührt. Man erhält eine Lösung mit einer Viskosität von 6,9 mPa·s und einem pH von 7,4. Die Lösung ist geeignet als Trägerlösung für ophthalmologische Präparate sowie als Tränenersatzmittel.

Beispiel 7

Rezeptur:

| 1. Polyethylenoxid, MG 5 Mio. | 1,000 kg |
|---|---|
| 2. Mannit | 18,000 kg |
| 3. Dinatrium-edetat | 0,040 kg |
| 4. Natriumsorbat | 0,800 kg |
| 5. Benzalkoniumchlorid | 0,040 kg |
| 6. dest. Wasser | 380,120 kg |

In einem 500-Liter-Reaktor mit Rührwerk und Doppelmantel werden die Bestandteile 2-5 im Bestandteil 6 gelöst. Anschließend erfolgt Zugabe des Bestandteils 1 und Auflösung desselben durch Rühren und Erwärmen auf 60-70°C. Die so bereitete Polymerlösung wird für 5 Minuten bei 134°C im Reaktor hitzesterlisiert, wobei das Polymer ausfällt. Der Reaktor wird unter Rühren abgekühlt, wodurch das Polymer wieder in Lösung geht. Man erhält eine Lösung mit einer Viskosität von 23,2 mPa•s und einem pH von 7,4. Die Losung ist geeignet als Trägerlösung für ophthalmologische Präparate sowie als Tränenersatzmittel.

**Patentansprüche**

1. Verwendung von Alkalisalzen der Sorbinsäure zur Verhinderung von Viskositätsverlust bei der Hitzesterilisierung wässeriger Lösungen, enthaltend Polyethylenoxid mit einem Molekulargewicht von 100.000 bis 5.000.000.

**Claims**

1. The use of alkaline salts of sorbic acid for preventing loss of viscosity when heat sterilizing aqueous solutions, containing polyethylene oxide with a molecular weight of 100,000 to 5,000,000.

**Revendications**

1. Utilisation de sel alcalin de l'acide sorbique destiné à empêcher la perte de viscosité lors de la stérilisation à la chaleur de solutions aqueuses contenant de l'oxyde de polyéthylène ayant un poids moléculaire de 100 000 à 5 millions.